Europäisches Patentamt

European Patent Office

Office européen des brevets

(19)

(11) Numéro de publication: **0 298 787**
**A1**

(12) **DEMANDE DE BREVET EUROPEEN**

(21) Numéro de dépôt: **88401363.2**

(22) Date de dépôt: **06.06.88**

(51) Int. Cl.⁴: **C 07 K 3/00**
// A61K35/50, A61K37/22

(30) Priorité: **12.06.87 FR 8708180**

(43) Date de publication de la demande:
**11.01.89 Bulletin 89/02**

(84) Etats contractants désignés:
**AT BE CH DE ES GB GR IT LI LU NL SE**

(71) Demandeur: **IMEDEX**
**58 avenue Leclerc**
**F-69007 Lyon (FR)**

(72) Inventeur: **Chirouze, Véronique**
**40 Chemin des Fonds**
**F-69110 Ste-Foy-Les-Lyon (FR)**

**Tayot, Jean-Louis**
**1 rue des Greffières**
**F-69890 La Tour De Salvagny (FR)**

(74) Mandataire: **Bernasconi, Jean et al**
**CABINET LEMOINE ET BERNASCONI 13, Boulevard des**
**Batignolles**
**F-75008 Paris (FR)**

(54) Procédé de préparation d'extraits lipidiques à haute teneur en phospholipides à partir de matériau placentaire.

(57) On procède à l'extraction simultanée de la quasi totalité des fractions lipidiques présentes dans le matériau à l'aide d'un solvant alcoolique polaire, notamment l'éthanol, l'extraction s'effectuant soit à partir d'une fraction tissulaire soit à partir d'une fraction non tissulaire. Le procédé permet de recueillir la quasi totalité des fractions lipidiques et notamment les phospholipides.

EP 0 298 787 A1

Bundesdruckerei Berlin

## Description

## Procédé de préparation d'extraits lipidiques à haute teneur en phospholipides à partir de matériau placentaire.

La présente invention a trait à un procédé de préparation d'extraits lipidiques à haute teneur en phospholipides à partir de matériau placentaire.

L'extraction de lipides à partir de tissus animaux ou humains s'effectue habituellement à l'aide de solvants peu polaires ou de mélanges d'un solvant peu polaire et d'un solvant très polaire, généralement un mélange chloroforme-méthanol ou mélange hexane-isopropanol. De tels procédés sont par exemple décrits dans la thèse d'ingénieur-docteur Véronique CHIROUZE, "Obtention d'un concentrat d'acide arachidonique à partir de placenta humain", Université de Bordeaux, 20 mai 1986. Ces procédés exigent de grands volumes de solvants très inflammables, relativement toxiques et demandant, par conséquent, des installations industrielles adaptées, lourdes et coûteuses.

Le brevet FR-A-2.575.385 décrit un procédé d'extraction de fractions lipidiques à partir de matériaux biologiques consistant à effectuer une protéolyse du matériau, suivie d'une extraction par solvant. Un tel procédé est complexe et le coût de ce traitement est augmenté à cause de l'étape de protéolyse.

La présente invention se propose de remédier à ces inconvénients et de fournir un procédé de préparation de lipides ou fractions lipidiques à haute teneur en phospholipides à partir de matériau placentaire, qui soit simple et efficace.

Un autre objectif de l'invention est de fournir un tel procédé qui permette d'extraire la quasi-totalité des lipides présents, y compris les lipides autres que phospholipides.

Un autre objectif de l'invention est de fournir un tel procédé qui soit non dénaturant et ne comporte pas d'apport de produits toxiques.

Un autre objectif encore de l'invention est de fournir un tel procédé permettant d'éliminer ou d'inactiver les particules virales ou acides nucléiques viraux.

Un autre objectif encore de l'invention est de fournir un tel procédé qui ne dénature pas le matériau traité et les composants de ce matériau de façon à permettre, le cas échéant, d'utiliser le matériau pour des opérations ultérieures de fractionnement, séparation ou purification, notamment de protéines.

L'invention a pour objet un procédé de préparation d'extraits lipidiques à haute teneur en phospholipides à partir de matériau placentaire, caractérisé en ce que l'on procède à l'extraction simultanée de la quasi-totalité des fractions lipidiques présentes dans le matériau à l'aide d'un solvant alcoolique polaire.

Par solvant alcoolique polaire, on entend principalement l'éthanol mais également d'autres alcools tels que le méthanol, l'isopropanol, l'alcool propylique.

Par matériau placentaire, on entend aussi bien le placenta brut que les fractions tissulaires ou non d'origine placentaire, y compris le sang placentaire, à condition qu'elles soient demeurées riches en phospholipides.

Conformément à l'invention, le solvant alcoolique, constitué de préférence d'un seul alcool, de préférence l'éthanol, est utilisé à l'état concentré et, de préférence, pratiquement pur.

Le procédé selon l'invention ne nécessite aucun traitement préalable ni digestion enzymatique et peut être mis en oeuvre directement sur le matériau placentaire, lequel peut avantageusement être soumis à un broyage grossier.

On constate, de façon surprenante, que le procédé selon l'invention permet non seulement de récupérer la quasi-totalité des phospholipides, et notamment des phospholipides riches en acides gras polyinsaturés à longue chaîne, mais également les autres fractions lipidiques, y compris les stéroïdes et les lipides neutres éventuellement présents.

Le pH pendant l'extraction alcoolique est pratiquement sans importance, des extractions ayant été réussies à des pH aussi acides que 3 ou aussi basiques que 9.

L'extraction s'effectue en utilisant un volume d'alcool de préférence au moins égal à trois fois le volume de matériau placentaire, et avantageusement d'environ cinq fois ce volume.

D'autres avantages et caractéristiques de l'invention apparaîtront à la lecture de la description suivante, faite à titre d'exemple non limitatif.

EXEMPLE 1

15 tonnes de placentas humains congelés sont broyées et décongelées à 10°C dans un volume de 10 m3 de solution contenant 6 g/l de NaCl à 8 % d'alcool éthylique. 5 tonnes de tissu placentaire sont récupérées par pressage. Dans le surnageant, les globulines du sang placentaire sont précipitées en bloc en 24 heures d'après la méthode de Taylor à pH neutre et à 5°C par addition d'alcool jusqu'à un taux de 25 % environ. Le tissu placentaire, pour sa part, est dispersé à pH 3,3 dans environ 25 000 litres d'une solution contenant 9 g/l de NaCl, à une température de 4°C. Le tissu acide est recueilli par pressage. Le tissu acide est ensuite dispersé dans 25 000 litres d'alcool éthylique à température ambiante et laissé en contact pendant 15 heures. Le tissu délipidé est éliminé par pressage puis soumis à un lavage éthanolique ultérieur puis à une digestion enzymatique permettant la préparation de différents collagènes d'origine placentaire. Le surnageant alcoolique est ensuite traité en vue d'obtenir un extrait lipidique débarrassé de toute substance indésirable, notamment de l'alcool présent, par exemple par évaporation.

La composition de la fraction lipidique obtenue est précisée dans le tableau 1 ci-dessous.

TABLEAU 1 : Composition des fractions
extraites du tissu placentaire acide
1) par l'alcool éthylique
2) par une technique classique

| Nature des lipides | kg lipides/5 tonnes tissus extraits à pH 3,3 par | |
|---|---|---|
| | Alcool éthylique | Technique classique |
| Phospholipides | 50,8 | 52 |
| Acides gras libres | 4,0 | 5,8 |
| Cholestérol libre | 14,2 | 17 |
| Cholestérol estérifié | 2,0 | 6,6 |
| Tryglycérides | 0,9 | 2,1 |
| Esters éthyliques | 0,1 | 0,1 |
| Lipides totaux | 72,0 kg | 83,6 kg |

Ces résultats montrent que l'extraction alcoolique permet de récupérer 86 % des lipides extraits par une technique classique (mélange hexane-isopropanol) mais 97 % des phospholipides. En effet, seuls les lipides les moins polaires (triglycérides, cholestérol estérifié) ne sont pas totalement extraits par l'alcool éthylique pur (ceci ne présente pas un inconvénient majeur car ces fractions lipidiques ne présentent pas en elles-mêmes un intérêt nutritionnel particulier). De plus, ce procédé d'extraction par l'alcool éthylique en milieu acide n'engendre pas la production notable de composés secondaires tels que les esters éthyliques.

Enfin, la composition en acides gras de la fraction majoritaire, les phospholipides, n'est pas modifiée par l'extraction par l'alcool éthylique pur. La composition en acides gras de cette fraction apparaît dans le tableau 2 ci-dessous.

3

TABLEAU 2 : Composition en acides gras de la fraction "phospholipides" extraite du tissu placentaire à pH acide

1) par l'alcool éthylique

2) par l'hexane/isopropanol

| | Extrait par | |
| --- | --- | --- |
| | ALCOOL ETHYLIQUE | HEXANE/ISOPROPANOL |
| Acides Gras | PHOSPHOLIPIDES | PHOSPHOLIPIDES |
| | moyenne | moyenne |
| 15:0 | -- | 0,33 |
| DMA 16:0 | 1,13 | 0,96 |
| DMA 16:1 | 1,24 | 3,68 |
| 16:0 | 22,09 | 18,10 |
| 16:1 n-7 | 1,41 | 1,15 |
| 17:0 | 0,48 | 0,44 |
| DMA 18:0 | 0,75 | 2,05 |
| DMA 18:1 | -- | 0,43 |
| 18:0 | 9,68 | 10,64 |
| 18:1 n-9 | 8,12 | 8,13 |
| 18:1 n-7 | 1,93 | 2,27 |
| 18:2 n-6 | 8,52 | 8,93 |
| 20:0 | -- | -- |
| 20:1 n-9 | -- | 0,24 |
| 20:2 n-6 | 0,45 | 0,52 |
| 20:3 n-6 | 4,97 | 5,23 |
| 20:4 n-6 | 26,88 | 24,19 |
| 22:0 | -- | -- |
| 22:1 n-9 | -- | -- |
| 22:4 n-6 | 2,73 | 2,76 |
| 22:5 n-6 | 2,02 | 2,09 |
| 22:5 n-3 | 0,96 | 1,32 |
| 22:6 n-3 | 5,59 | 5,05 |
| autres | | |

EXEMPLE 2 - Tissu brut pH 4,5

Le tissu placentaire pressé de l'exemple 1 est dispersé à pH 4,6 dans environ 25.000 litres d'un tampon acétate, recueilli par pressage puis dispersé dans 17.500 litres d'alcool éthylique pur à température ambiante et laissé en contact pendant 15 heures. Le tissu délipidé est éliminé par pressage et le surnageant alcoolique traité pour obtenir un extrait lipidique convenable.

La composition de l'extrait lipidique apparaît dans le tableau 3 ci-dessous.

TABLEAU 3 : Composition des fractions extraites du tissu placentaire acide

1) par l'alcool éthylique

2) par une technique classique

| Nature des lipides | kg lipides/5 tonnes tissus extraits à pH 4,5 par | |
| --- | --- | --- |
| | Alcool éthylique | Technique Classique |
| Phospholipides | 38,8 | 40,1 |
| Acides gras libres | 8,5 | 10,1 |
| Cholestérol libre | 16,0 | 19,1 |
| Cholestérol estérifié | 2,1 | 4,8 |
| Triglycérides | 0,8 | 1,6 |
| Esters éthyliques | 0,1 | 0,1 |
| Lipides totaux | 66,3 kg | 75,8 kg |

L'extraction alcoolique permet de récupérer 87 % des lipides extraits par une technique clasique (mélange hexane-isopropanol) mais 97 % des phospholipides.

EXEMPLE 3.

Le précipité de globulines de l'exemple 1 est recueilli par centrifugation puis dispersé à pH 4,8 dans environ 10.000 litres d'une solution contenant 4 g/l de chlorure de sodium, à une température de 2 °C. Environ 1.200 l d'insoluble, appelé précipité B1, sont recueillis par centrifugation alors que le surnageant est soumis à un fractionnement ultérieur permettant la préparation des gammaglobulines. Le pH du précipité B1 est ajusté à 3 par addition d'acide chlorhydrique concentré. Le précipité B1 acidifié est ensuite dispersé dans 6.000 l d'alcool éthylique et laissé en contact pendant 15 heures à température ambiante. Le précipité B1 délipidé est éliminé par centrifugation. Le surnageant alcoolique est traité de façon à obtenir un extrait lipidique convenable.
La composition de l'extrait lipidique obtenu est indiquée dans le tableau 4 ci-dessous.

TABLEAU 4 : Composition des fractions extraites du précipité B1 acide par

1) l'alcool éthylique
2) l'hexane-isopropanol

| Nature des lipides | kg lipides/1200 l PRECIPITE B1 extraits à pH 3 par | |
|---|---|---|
| | alcool éthylique | hexane/isopropanol |
| Phospholipides | 13,2 | 12,8 |
| Acide gras libres | 2,7 | 2,8 |
| Cholestérol libre | 4,5 | 6,1 |
| Cholestérol estérifié | 1,8 | 6,6 |
| Tryglycérides | 1,2 | 2,3 |
| Esters éthyliques | 0,8 | 0,8 |
| Lipides totaux | 24,2 | 31,4 |

La fraction éthanolique contient 77 % des lipides extraits par l'hexane-isopropanol mais la totalité des phospholipides sont extraits du précipité B1 par l'éthanol seul. De plus la composition en acides gras de cette fraction "phospholipides" est la même quelle que soit la technique d'extraction utilisée.

Des résultats semblables sont obtenus par extraction éthanolique selon l'invention à pH basique, par exemple en tampon carbonate.

Les différents lipides obtenus peuvent être ensuite purifiés et fractionnés de façon usuelle.

**Revendications**

1. Procédé de préparation d'extraits lipidiques à haute teneur en phospholipides à partir de matériau placentaire, caractérisé en ce que l'on procède à l'extraction simultanée de la quasi-totalité des fractions lipidiques présentes dans le matériau à l'aide d'un solvant alcoolique polaire.

2. Procédé selon la revendication 1, caractérisé en ce que l'on effectue l'extraction par l'éthanol.

3. Procédé selon la revendication 1, caractérisé en ce que l'on effectue l'extraction à l'aide d'un autre alcool tel que le méthanol, l'isopropanol, l'alcool propylique.

4. Procédé selon l'une quelconque des revendications 1 à 3, caractérisé en ce que l'on effectue l'extraction avec un volume d'alcool au moins égal à trois fois le volume de matériau placentaire, et notamment d'environ 5 fois ce volume.

5. Procédé selon l'une quelconque des revendications 1 à 4, caractérisé en ce que l'on effectue l'extraction à partir d'une fraction tissulaire d'origine placentaire.

6. Procédé selon la revendication 5, caractérisé en ce que le tissu placentaire récupéré par pressage après séparation du sang placentaire, est dispersé dans une solution saline puis pressé, après quoi le tissu est dispersé dans l'alcool éthylique, le tissu est éliminé par pressage et le surnageant alcoolique, qui contient les lipides et notamment les phospholipides, est recueilli.

7. Procédé selon l'une quelconque des revendications 1 à 4, caractérisé en ce que l'on effectue l'extraction d'une fraction d'origine non tissulaire du placenta.

8. Procédé selon la revendication 7, caractérisé en ce que l'on effectue l'extraction sur le bloc de globulines obtenu à partir du sang placentaire.

9. Procédé selon la revendication 8, caractérisé en ce que l'on disperse le bloc de globulines dans une solution saline, après quoi l'on recueille l'insoluble (précipité B1), on acidifie l'insoluble et on le disperse dans l'alcool, l'on élimine le précipité subsistant, et l'on recueille le surnageant contenant les fractions lipidiques, et notamment les phospholipides.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 537 585 (EMPRESA CUBANA IMPORTADORA Y EXPORTADORA DE PRODUCTOS MEDICOS)<br>* Page 3, lignes 24-29; revendications 1,2 * | 1-9 | C 07 K 3/00 //<br>A 61 K 35/50<br>A 61 K 37/22 |
| Y | WO-A-8 604 354 (JAP. DIRECTOR GENERAL OF AGENCY OF INDUSTRIAL SCIENCE AND TECHNOLOGY)<br>* Page 3, lignes 19-24; page 3, ligne 30 - page 4, ligne 15; page 6, lignes 1-8,25-33; page 9, lignes 8-13; revendications 1,2 * | 1-9 | |
| A | CHEMICAL ABSTRACTS, vol. 107, no. 20, 16 novembre 1987, page 481, résumé no. 183400a, Columbus, Ohio, US; V. CHIROUZE et al.: "Human placenta as a source of long-chain polyunsaturated fatty acids. I. Placental lipid composition and potential yields of arachidonic acid", & REV. FR. CORPS GRAS 1987, 34(5-6), 275-80<br>* Résumé * | 1-9 | DOMAINES TECHNIQUES RECHERCHES (Int. Cl.4)<br><br>A 61 K |
| A | BIOLOGICAL ABSTRACTS, vol. 83, no. 3, 1987, résumé no. 27893, Philadelphia, US; K.S. MUKHAMEDOVA et al.: "Phospholipids from the ripening seeds of Sophora japonica", & KHIM. PRIR. SOEDIN (TASHK), 0(3), 281-284, 1986<br>* En entier * | 1,3 | |
| | --- -/- | | |

Le présent rapport a été établi pour toutes les revendications

| Lieu de la recherche | Date d'achèvement de la recherche | Examinateur |
|---|---|---|
| LA HAYE | 27-09-1988 | CHARLES D.J.P.I.G. |

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 388 823 (A. NATTERMANN & CIE GmbH) <br> * Page 1, lignes 1-13; page 3, ligne 32 - page 4, ligne 4; page 5, lignes 7-13; revendication 1 * <br> ----- | 1 | |

**DOMAINES TECHNIQUES RECHERCHES (Int. Cl.4)**

Le présent rapport a été établi pour toutes les revendications

| Lieu de la recherche | Date d'achèvement de la recherche | Examinateur |
|---|---|---|
| LA HAYE | 27-09-1988 | CHARLES D.J.P.I.G. |